# EUROPEAN PATENT SPECIFICATION

(11) **EP 4 099 921 B1**
(45) Date of publication and mention of the grant of the patent: **31.01.2024**
(21) Application number: 21704447.8
(22) Date of filing: 04.02.2021
(51) Int. Cl.: A61B 17/12

(54) **EMBOLISATION SYSTEM FOR PROMOTING CLOT FORMATION**
EMBOLISATIONSSYSTEM ZUR FÖRDERUNG DER GERINNSELBILDUNG
SYSTÈME D'EMBOLISATION POUR FAVORISER LA FORMATION DE CAILLOT

(30) Priority: 04.02.2020 WO PCT/EP2020/052757
(43) Date of publication of application: 14.12.2022
(73) Proprietor: Clearstream Technologies Limited, Enniscorthy, County Wexford (IE)
(72) Inventor: KAVANAGH, Michaella, Enniscorthy, County Wexford (IE)
(74) Representative: Hoffmann Eitle
(86) International application number: PCT/EP2021/052625
(87) International publication number: WO 2021/156343

(56) References cited:
- US-A1- 2009 062 838
- US-A1- 2012 022 572
- US-A1- 2015 173 770
- US-A1- 2016 166 257

## Description

### Technical field

The present disclosure relates to an embolisation device for promoting clot formation in a bodily lumen, comprising an embolisation device having a contracted delivery configuration and an expanded deployed configuration for anchoring the embolisation device in the bodily lumen. The present disclosure also relates to a method of manufacturing an embolisation device.

### Background

Embolisation devices may be deployed in the vasculature at a particular location by a medical practitioner so as to promote blood clot formation and ultimately occlude the blood vessel. Typically, an embolisation device is pushed through a guide (delivery) catheter in a distal direction using a delivery wire until a point of deployment within a bodily lumen is reached. Once the device reaches the required point of deployment, the device is deployed from the guide catheter.

The embolisation device has a contracted delivery configuration as it is pushed through the delivery catheter, and expands to an expanded deployed configuration when the device is deployed from the guide catheter and is positioned in the bodily lumen. The embolisation device has a larger radial profile in the expanded deployed configuration than in the contracted delivery configuration. The embolisation device may be recaptured by the delivery catheter and may therefore return to the contracted delivery configuration from the expanded deployed configuration, before being redeployed in the bodily lumen.

The embolisation device therefore moves between the contracted delivery configuration and the expanded deployed configuration, in some cases multiple times.

There exists a need for the various components of the embolisation device to move between the two configurations without reducing the performance of the element. For example, there exists a need for the vessel occlusion rate of the embolisation device not to deteriorate after being deployed or even re-captured and re-deployed.

US 2016/166257 A1 discloses a bristle device for delivery into a body lumen comprising a longitudinally extending stem and a plurality of bristles extending generally outwardly from the stem for anchoring the device in a body lumen. There may be at least two bristle segments and in some cases there are flexible sections between the segments. The flexible sections articulate to enable the device to pass through a catheter placed in a tortuous anatomy or to be deployed in a curved vessel, or across a bifurcation. In some cases at least some of the bristle segments are spaced-apart to accommodate bending of the bristles.

US 2012/022572 A1 discloses a device for protecting cerebral vessels or brain tissue during treatment of a carotid vessel including a catheter having a distal portion, a proximal portion and a lumen extending therebetween, the catheter including first and second expandable areas provided over the length of the catheter. The device includes a first elongate member insertable longitudinally through the lumen of the catheter, the first elongate member being configured for stretching at least a portion of the catheter and causing one of the first and second expandable areas to transition from an expanded state to a collapsed state. The device further includes a second elongate member insertable longitudinally through the lumen of the catheter, the second elongate member being configured for stretching at least a portion of the catheter and causing the other of the first and second expandable areas to transition from an expanded state to a collapsed state.

### Summary

According to a first aspect, there is provided embolisation device for promoting clot formation in a bodily lumen, comprising a stem and a plurality of flexible bristles extending outwardly from the stem, the bristles having a contracted delivery configuration and an expanded deployed configuration in which the bristles extend at least radially outwardly from the stem to anchor the device in the lumen, wherein the embolisation device further comprises: a flow restrictor configured to restrict flow through the bodily lumen, the flow restrictor comprising: a membrane support having a contracted delivery configuration and an expanded deployed configuration and comprising a self-expanding mesh extending at least radially outwardly from the stem; and an occluding mesh membrane mounted on the membrane support wherein the occlusion rate of the occluding mesh membrane is greater than that of the membrane support; wherein the flow restrictor is attached to the stem at a first longitudinal end, the flow restrictor further comprising an open second longitudinal end, and wherein the occluding membrane is configured to restrict flow through the lumen when the device is deployed and the membrane support is in the expanded deployed configuration. As a higher occlusion rate of a membrane generally affects the ability of the membrane to expand from a contracted delivery configuration to an expanded delivery configuration (for example due to tearing or unwanted folding), the provision of a membrane support allows for an occluding membrane with improved occluding properties to be selected. The flow restrictor being attached to the stem at a first longitudinal end and having an open second longitudinal end provides a flow restrictor with excellent anchoring properties for a particular bloodflow direction. The term "open" may be understood to mean that, in the expanded deployed configuration, the membrane support and the occluding mesh membrane extend circumferentially about the stem such that bloodflow may pass through the second longitudinal end towards the first longitudinal end. The flow restrictor is closed at the first longitudinal end, meaning that bloodflow is restricted from passing through the first longitudinal end of the flow restrictor.

The occluding membrane may be a metallic mesh membrane, such as Nitinol. The metallic mesh membrane has a greatly reduced risk of tearing when compared with a polymer membrane.

The mesh may be attached to the stem at a first longitudinal end, the mesh further comprising a tubular section extending longitudinally along the stem, wherein the tubular section is configured to contact the bodily lumen in the expanded deployed configuration. The mesh provides extensive support to the occluding membrane across the surface area of the occluding membrane. The tubular section contacts the bodily lumen along a length thus providing a larger anchoring force to the bodily lumen.

The membrane may be mounted radially outside of the self-expanding mesh. This configuration may be simpler to manufacture.

The occluding membrane may comprise a first network of wires having a first average diameter, and the self-expanding mesh may comprise a second network of wires having a second average diameter, wherein the second average diameter is greater than the first average diameter. The greater diameter wires of the mesh provides mechanical strength to the occluding membrane.

The occluding membrane may comprise a plurality of pores, wherein the number of pores per unit area of the occluding membrane may be greater than the number of pores per unit area of the self-expanding mesh. The self-expanding mesh acts as a scaffold for the occluding membrane and provides mechanical support to the occluding membrane.

The occluding membrane may comprise a plurality of pores having a lateral extent of less than 200µm and preferably less than 100µm. That is to say, the extent of the pores may be less than 200µm and preferably less than 100µm in a given direction across the pore. This range of pore sizes provides low porosity thus preventing blood flow through the device.

The embolisation device may further comprise a tubular connector receiving the stem and the membrane support to attach the membrane support to the stem. The occluding membrane may be attached to the stem by the tubular connector. The tubular connector may be a crimping connector. The use of a tubular connector or a crimping connector may simplify the manufacture of the embolisation device.

The occluding membrane may be attached to the membrane support by adhesive or welding.

The membrane support and occluding membrane may be spaced apart from the plurality of bristles such that in the expanded deployed configuration the membrane support and membrane are free from contact with any bristles. The membrane support in this configuration may engage the lumen with sufficient anchoring force such that only one bristle segment is required to anchor the device to the lumen.

The embolisation device may be configured to be delivered from a delivery catheter to the bodily lumen in a distal direction, and comprise a bristle segment positioned distally from the flow restrictor. The membrane support in this configuration may provide sufficient anchoring force in the bodily lumen such that a proximal bristle segment is not required for the embolisation device.

The embolisation device may comprise a bristle segment and the flow restrictor may be located longitudinally within the bristle segment or directly adjacent the bristle segment.

The embolisation device may be configured to be delivered from a delivery catheter to the bodily lumen in a distal direction, the bristle segment may be a proximal bristle segment and the embolisation device may further comprise a distal bristle segment spaced apart in the distal direction from the proximal bristle segment (i.e. the spacing between the segments being greater than the spacing between the bristles within a segment).

According to a second aspect, there is provided a method of manufacturing an embolisation device for promoting clot formation in a bodily lumen, comprising: providing a stem; providing a plurality of flexible bristles extending outwardly from the stem, the bristles having a contracted delivery configuration and an expanded deployed configuration in which the bristles extend at least radially outwardly from the stem to anchor the device in the lumen; providing a membrane support having a contracted delivery configuration and an expanded deployed configuration and comprising a self-expanding mesh; mounting an occluding mesh membrane on the membrane support; and attaching the membrane support to the stem such that it extends at least radially outwardly from the stem, the membrane support having a contracted delivery configuration and an expanded deployed configuration in which the radial profile of the membrane support is greater than in the contracted delivery configuration; wherein the membrane support is attached to the stem at a first longitudinal end, the membrane support further comprising an open second longitudinal end, and the occluding membrane is configured to restrict flow through the lumen when the device is deployed and the membrane support is in the expanded deployed configuration.

The steps of providing the membrane support, mounting the occluding membrane on the membrane support and attaching the membrane support to the stem may comprise the steps of: providing a self-expanding tubular mesh; wrapping the occluding membrane around, and attaching it to, the tubular mesh; providing a tubular connector on the stem; inserting an end of the tubular mesh into the tubular connector; and crimping the connector. The manufacturing method may be simplified and more reliable by using a crimp connector to connect the tubular mesh to the stem.

The occluding membrane may be wrapped around and attached to the tubular mesh before the tubular mesh is connected to the stem by the crimping connector. This may simplify the process of attaching the membrane to the tubular mesh.

### Brief description of the drawings

To enable a better understanding of the present disclosure, and to show how the same may be carried into effect, reference will now be made, by way of example only, to the accompanying schematic drawings, in which:
FIGS. 1A shows a side view of an embolisation device according to one or more embodiments of the present disclosure at various stages of the delivery process of the embolisation device to a bodily lumen;
FIGS. 1B shows another side view of an embolisation device according to one or more embodiments of the present disclosure at various stages of the delivery process of the embolisation device to a bodily lumen;
FIGS. 1C shows another side view of an embolisation device according to one or more embodiments of the present disclosure at various stages of the delivery process of the embolisation device to a bodily lumen;
FIG. 2A shows another side view of another embolisation device according to one or more embodiments of the present disclosure;
FIG. 2B shows a cross sectional view of the flow restrictor of the embolisation device of FIG. 2A across the line A-A according to one or more embodiments of the present disclosure;
FIG. 3 shows a side view of an embolisation device according to one or more embodiments of the present disclosure; and
FIG. 4 shows a side view of another embolisation device according to one or more embodiments of the present disclosure.

### Detailed description

Throughout this disclosure the term `embolisation device' may refer to a device which may be permanently or semi-permanently implanted in a bodily lumen. Accordingly, the `embolisation device' may be configured to be disposed within the bodily lumen for a period of time, such as a number of days, or disposed in the lumen indefinitely. To this end, the `embolisation device' may be configured to be selectively detached from a delivery element so that it may be implanted in the bodily lumen in isolation.

Throughout this disclosure the term `bodily lumen' may refer to the inside space within a tubular structure of the human or animal body. The `bodily lumen' may be, for example, an artery or vein.

Throughout this disclosure the term `contracted delivery configuration' of an element may refer to a configuration of the element which has a smaller radial extent than an expanded deployed configuration of the element.

Throughout this disclosure the term 'to anchor' may refer to partly or fully securing an element in a position.

Throughout this disclosure, the term 'stem' may refer to an elongate element which extends longitudinally along the length of the embolisation device to act as a backbone for the device, and has a significantly smaller radial extent than the further elements of the embolisation device (for example, the plurality of flexible bristles). The stem may extend along substantially the whole longitudinal extent of the plurality of flexible bristles (e.g. when the embolisation device is in an unrestrained configuration, contracted delivery configuration and/or expanded deployed configuration). The stem may extend along substantially the whole length of the embolisation device.

In any of the examples described herein, the term 'bristle' may refer to an elongate strand of material formed substantially a single piece. The 'bristle' may be a resilient bristle. The resilient bristle may be biased towards a particular curvature.

Throughout this disclosure, the term `radially outwardly' does not exclude the element additionally extending in the longitudinal direction of the device. For example, the plurality of flexible bristles may extend radially outwardly and longitudinally from the stem.

Through this disclosure, the term "radial profile" may refer to a radial extent in a particular direction radially outwardly from the stem of the embolisation device. For example, the embolisation device has a lower radial extent in the contracted delivery configuration than in the expanded deployed configuration.

The plurality of flexible bristles may have a contracted configuration in the contracted delivery configuration. The plurality of flexible bristles may have an expanded configuration in the expanded deployed configuration. The plurality of bristles may extend radially outwardly from the stem in a plurality of circumferential directions about the stem.

In the expanded configuration, the plurality of flexible bristles may be configured to anchor the device in the bodily lumen. The plurality of flexible bristles may be configured to provide part or substantially all of the anchoring force for the embolisation device in the bodily lumen. The membrane support may additionally be configured to provide part of the anchoring force for the embolisation device in the bodily lumen.

In the expanded configuration, the plurality of flexible bristles may be configured to contact the bodily lumen.

In the contracted delivery configuration, the plurality of flexible bristles extending outwardly from the stem are contracted such that radial extent of the device is less than the radial extent of the expanded deployed configuration of the element. In the contracted delivery configuration the device fits inside the delivery catheter.

The bristles may be made of a flexible or resiliently deformable material such as stainless steel, Elgiloy or Nitinol. Other suitable materials may also be used, such as any suitable polymer or any other shape memory metal or metal alloy. The membrane may be a metallic mesh membrane made of materials such as stainless steel or Nitinol. The membrane support may be made of any self-expanding material, such as a polymer, Nitinol or stainless steel. The stem may be made of stainless steel or other suitable material and may comprise a twisted wire from which the bristles extend and on which the flow restrictor is mounted. The stem may alternatively comprise a hollow tube wherein the walls of the hollow tube hold the radially extending bristles in place. For example, the tube may be formed from a heat shrinkable material as is well known in the art. Alternatively or additionally, the bristles may be held by the stem using other means such as adhesives.

The diameter of an individual flexible bristle may range from 0.036mm (0.0014 inches) to 0.053mm (0.0021 inches). For example, the diameter of an individual flexible bristle may be 0.381mm (0.015 inches), 0.445mm (0.0175 inches) or 0.508mm (0.02 inches). The overall radial diameter of the expanded flexible bristles may range from 5mm to 30mm.

The occluding membrane may be a thin film membrane. For example, the occluding membrane may have a thickness of 4µm to 35µm and a radial diameter, in the deployed configuration, of 5mm to 20mm. For example, the diameter of the membrane may be 6.5mm, 9mm or 16mm.

In the illustrated embodiments described herein, the same reference numerals are used for corresponding elements in each example.

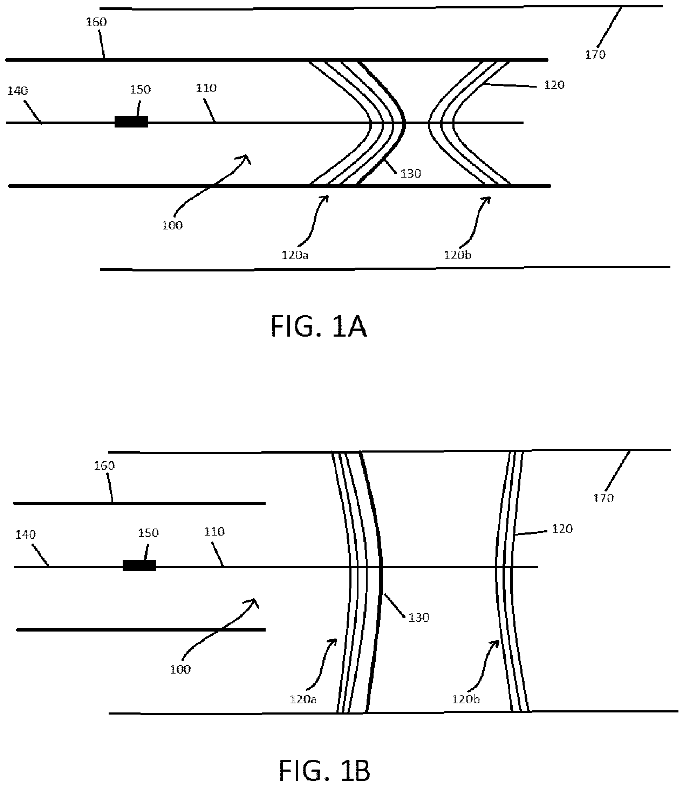

Figure 1A shows an embolisation device 100 situated inside a delivery catheter 160. The delivery catheter 160 is used to guide the embolisation device 100 through the delivery catheter 160 in a proximal direction towards a target site within a bodily lumen 170. The embolisation device comprises a stem 110 and a plurality of bristles 120 extending generally (i.e. at least) radially outwardly from the stem 110. In Figure 1A, the plurality of bristles are in a contracted delivery configuration inside the delivery catheter 160. The embolisation device 100 further comprises a flow restrictor comprising an occluding membrane 130 mounted on a self-expanding membrane support (not shown).

In some examples, the plurality of bristles 120 may all extend radially outwardly and longitudinally distally in the contracted delivery configuration, or longitudinally proximally in the contracted delivery configuration, or combination of the two. In the illustrated example, the plurality of bristles 120 are divided into a proximal bristle segment 120a and a distal bristle segment 120b. The bristles of the proximal bristle segment 120a extend radially outwardly and longitudinally proximally in the contracted delivery configuration, whereas the bristles of the distal bristle segment 120b extend radially outwardly and longitudinally distally in the contracted delivery configuration. Such a configuration effectively anchors the device to the bodily lumen once deployed.

In the illustrated example, the flow restrictor is situated adjacent to the distal end of the proximal bristle segment 120a, whereas in other examples it may be situated elsewhere, such as, for example, longitudinally within one of the bristle segments, adjacent to the proximal end of the proximal bristle segment 120a or adjacent to the proximal or distal ends of the distal bristle segment 120b.

The occluding membrane has greater occluding properties than the membrane support. In other words, the occluding membrane causes a greater rate of occlusion in the vessel than the membrane support. Due to the presence of the membrane support, the occluding rate of the occluding membrane may be selected to be high without compromising on the required structural strength of the flow restrictor, as this structural strength is provided by the membrane support.

The occluding membrane is a mesh membrane. The use of a mesh membrane provides a structure for cell attachment an growth and thus increase occlusion of the device. Reducing the mesh pattern size (i.e. increasing the number of pores per unit area) increases the blood contact surface area to facilitate increased occlusion rates. This is especially true of micromesh patterns that can be achieved by using thin film membranes (i.e. membranes having a thin film thickness).

Some devices may use a polymer membrane. Polymer membranes are prone to tearing during manufacturing or movement of the device between the contracted delivery configuration and the expanded deployed configuration, reducing the effectiveness of the device, especially when recaptured and redeployed. Some devices may have the membrane positioned adjacent a bristle segment or longitudinally within a bristle segment. The neighbouring bristles will guide the membrane passively between the contracted delivery configuration and the expanded deployed configuration. However, especially for thin film membranes, the passive force of the neighbouring bristles may not reliably move the thin film membrane between the contracted delivery configuration and the expanded deployed configuration (i.e. may not provide adequate support to the membrane to fold and unfold the membrane in a reliable manner, which may in turn affect occlusion of the device).

The embolisation device is guided through the delivery catheter 160 using the delivery element 140. The delivery element 140 is connected to a proximal end of the embolisation device 100 via a releasable connecting mechanism 150.

Once the embolisation device has been delivered to the target site within the bodily lumen 170, then the embolisation device is moved distally relative to the delivery catheter 160 such that the bristles 120 expand to the expanded deployed configuration and engage the walls of the bodily lumen 170 to anchor the device to the lumen 170. This configuration is shown in Figure 1B.

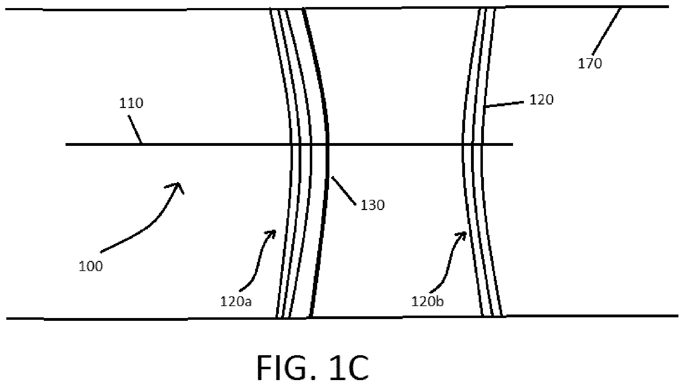

If it is determined that the embolisation device is incorrectly placed within the bodily lumen 170, then it may be recaptured by the delivery catheter 160 and redeployed at a new position within the bodily lumen 170. Once it is determined that the final placement of the embolisation device 100 is correct, the embolisation device 100 is released from the delivery element 140 and the device 100 is implanted in the bodily lumen 170. This is shown in Figure 1C.

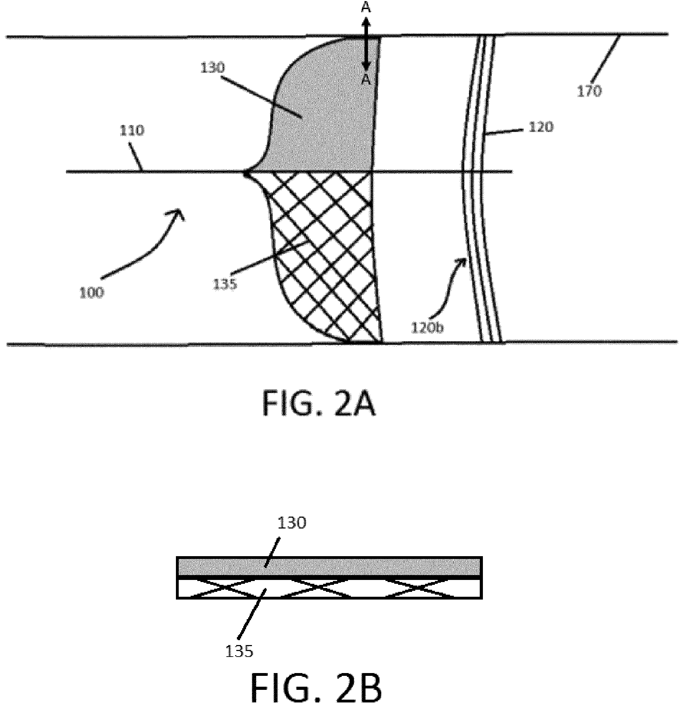

Figure 2A shows another exemplary embolisation device 100 according to the present disclosure. The device 100 is shown in the expanded deployed configuration, similar to that shown in Figure 1C. Similar to the device shown in Figures 1A to 1C, the embolisation device 100 comprises a plurality of bristles 120 extending radially outwardly from the stem 110. The device 100 shown in Figure 2A is delivered to a bodily lumen in the same manner as described with reference to Figures 1A to 1C. Whilst in the illustrated example the bristles 120 are comprised in a single segment 120b, the bristles extending distally in a longitudinal direction, it will be apparent to the skilled person that other configurations of bristles are used in other examples.

In the example shown in Figure 2A, the plurality of bristles 120 are spaced apart from the occluding membrane 130 such that in the expanded deployed configuration the membrane support and the membrane do not contact any bristles.

The occluding membrane 130 is mounted on a self-expanding membrane support 135. The occluding membrane 130 is a mesh membrane and the membrane support 135 comprises a self-expanding mesh onto which the occluding membrane is mounted. In the device 100 shown in Figure 2A, the occluding membrane 130 is mounted on the outside of the membrane support 135. The membrane 130 is omitted from the lower half of the schematic of Figure 2A to illustrate the membrane support 135 radially inward of the membrane 130. The membrane 130 may be attached to the membrane support 135 by any attaching means, such as, for example, by adhesive or welding.

The flow restrictor, which comprises the occluding membrane 130 and the membrane support 135, is attached to the stem at a first longitudinal end (the left end shown in Fig. 2A). At a second longitudinal end opposite the first longitudinal end, the flow restrictor is open, meaning that the membrane support 135 and membrane 130 are both located radially away from the stem and extend circumferentially about the stem. Accordingly, bloodflow is able to pass through the second longitudinal end towards the first longitudinal end. The first longitudinal end of the flow restrictor is closed, meaning bloodflow is restricted from flowing past the first longitudinal end. When blood flows in this direction, pressure is exerted outwardly on the parts of the flow restrictor contacting the wall of the lumen 170, thus increasing the anchoring properties of the flow restrictor. Accordingly, providing a flow restrictor which is open at one longitudinal end provides increased anchoring for bloodflow in one direction.

Figure 2B shows a cross-sectional view of the device shown in Figure 2A across the line A-A depicting the occluding membrane 130 mounted on the radial outer side of the membrane support 135. In other examples, the membrane 130 may be mounted on the radial inner side of the membrane support 135.

The membrane support 135 may be any self-expanding structure that is suitable for moving the occluding membrane 130 between the contracted delivery configuration and the expanded deployed configuration. The membrane support 135 comprises a self-expanding mesh. The mesh membrane support provides support across the surface area of the membrane 130 and ensures that the membrane 130 moves between the contracted delivery configuration and the expanded deployed configuration in a reliable manner. As illustrated in Figure 2A, the membrane support 135 comprises a self-expanding mesh. The self-expanding mesh is mounted on the stem at a first longitudinal end of the and further comprises a tubular section extending longitudinally along the stem from the first longitudinal end. The tubular section may be configured to have a greater diameter along its length, in a relaxed, unconstrained configuration, than the bodily lumen 170, so that the flow restrictor abuts the bodily lumen 170 in the deployed configuration as shown in Figure 2A. the membrane 130 is mounted radially outside of the self-expanding mesh (whereas in other examples the membrane 130 may be mounted on the self-expanding mesh on the radial inner side). The membrane support 135 may be mounted on the stem by any suitable means, such as by welding or adhesive. The membrane 130 may also be directly mounted on the stem, or may be mounted on the stem via the membrane support 135. Whilst in the illustrated example the first longitudinal end of the self-expanding mesh (i.e. the mounting point of the mesh on the stem) is positioned proximally to the tubular section, in other examples the mounting point may be positioned distally to the tubular section. It will be understood by the skilled person that the longitudinal length of the tubular section may be selected as appropriate for a given embolisation device.

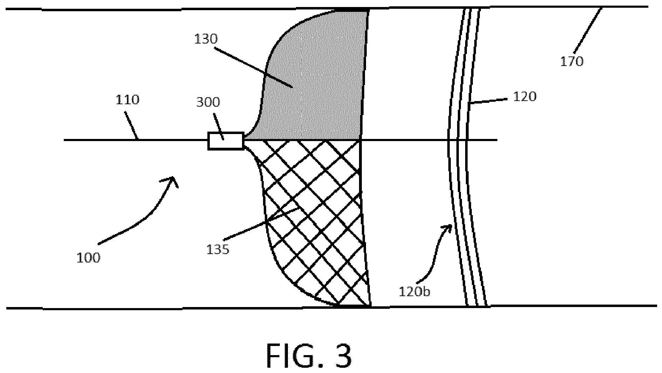

Figure 3 shows a further exemplary embolisation device 100 according to the present disclosure. The device 100 is similar to the device shown in Figure 2A, except for the manner in which the membrane support 135 is mounted on the stem 110. Accordingly, the variants disclosed in relation to Figures 2A and 2B with respect to the various alternative configurations of the bristles 120, membrane 130 and 135 are also contemplated with the device shown in Figure 3. Namely, other configurations of bristles to that illustrated are used in other examples; the membrane 130 may be attached to the membrane support by any attaching means, such as, for example, by adhesive or welding; the membrane 130 may be mounted on the radial inner side of the membrane support 135; the membrane support 135 may be any self-expanding structure that is suitable for moving the occluding membrane 130 between the contracted delivery configuration and the expanded deployed configuration; the membrane support 135 comprises a self-expanding mesh; and the mounting point of the self-expanding mesh may be positioned distally to the tubular section of the mesh.

Whilst in the examples disclosed with respect to Figures 2A and 2B the membrane support 135 (and optionally the membrane 130) is mounted on the stem by welding or adhesive, in the device 100 of Figure 3 the membrane support 135 (and optionally the membrane 130) is mounted on the stem 110 by a tubular connector 300. The tubular connector 300 may be a crimping connector. In manufacturing the device 100, the membrane support 135 (and optionally the membrane 130) may be inserted into the connector 300 (the connector 300 receiving the stem 110) and the connector 300 may then be crimped to securely fasten the membrane support 135 (and optionally the membrane 130) to the stem 110.

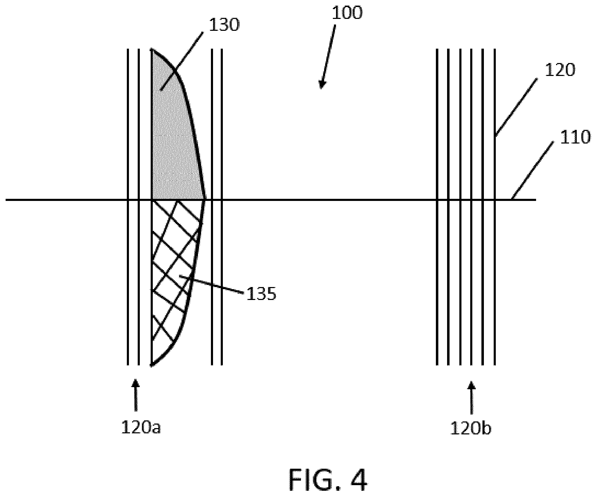

Figure 4 shows a further exemplary embolisation device 100 according to the present disclosure. The device 100 is shown in an unconstrained configuration. Similar to the devices shown in Figures 1A to 3, the embolisation device 100 comprises a plurality of bristles 120 extending radially outwardly from the stem 110. The device 100 shown in Figure 4 is delivered to a bodily lumen in the same manner as described with reference to Figures 1A to 1C. As in the case of the device shown in Figures 1A to 1C, the device 100 comprises a proximal bristle segment 120a and a distal bristle segment 120b. The device further comprises an occluding membrane 130 mounted on a self-expanding membrane support 135. In the present example, the membrane 130 is located longitudinally within the bristles of the proximal bristle segment 120a. The membrane 130 may be attached to the membrane support by any attaching means, such as, for example, by adhesive or welding, or by a crimping connector similar to that shown in Figure 3. The membrane 130 may be mounted on the radial outer side or radial inner side of the membrane support 135.

As in the examples shown in Figures 2 and 3, The membrane support 135 may be any self-expanding structure that is suitable for moving the occluding membrane 130 between the contracted delivery configuration and the expanded deployed configuration, and comprises the self-expanding meshes previously described. In the example shown in Figure 4, the support 135 comprises a self-expanding mesh mounted on the stem at a first longitudinal end. In the illustrated example, the first longitudinal end of the tubular mesh is positioned distally to the tubular section of the self-expanding mesh, whilst in other examples the first longitudinal end may be positioned proximally to the tubular section of the self-expanding mesh.

In yet further examples, the occluding membrane 130 shown in Figure 4 may be located longitudinally adjacent to the proximal segment 120a or the distal segment 120b. For example, the membrane 130 may be placed longitudinally adjacent to the proximal segment 120a on the proximal side or the distal side of the proximal segment 120a, or longitudinally adjacent to the distal segment 120b on the proximal side or the distal side of the distal segment 120b.

The various embolisation devices 100 according to the present disclosure can be manufactured by the following method:
- providing a stem;
- providing a plurality of flexible bristles extending outwardly from the stem, the bristles having a contracted delivery configuration and an expanded deployed configuration in which the bristles extend at least radially outwardly from the stem to anchor the device in the lumen;
- providing a self-expanding membrane support;
- mounting an occluding membrane on the membrane support; and
- attaching the membrane support to the stem such that it extends at least radially outwardly from the stem, the membrane support having a contracted delivery configuration and an expanded deployed configuration in which the radial profile of the structure is greater than in the contracted delivery configuration;
- wherein the occluding membrane is configured to restrict flow through the lumen when the device is deployed and the membrane support is in the expanded deployed configuration.

It is noted that the steps of the above method are not necessarily presented in chronological order. For example, the bristles could be provided on the stem before or after the membrane support is attached to the stem. Likewise, the membrane support could be attached to the stem before or after the occluding membrane is mounted on the membrane support. Other variants will be apparent to the skilled person.

In some examples, the method may comprise the steps of:
- providing a self-expanding tubular mesh;
- wrapping the occluding membrane around, and attaching it to, the tubular self-expanding mesh;
- providing a tubular connector on the stem;
- inserting an end of the tubular mesh into the tubular connector; and
- crimping the connector.

It is again noted that the steps are not necessarily presented in chronological order. For example, the membrane could be wrapped around and attached to the tubular mesh before or after and end of the tubular mesh is inserted into the tubular connector, and before or after the connector is crimped. Similarly, the tubular connector could be provided on the stem before or after an end of the tubular mesh is inserted into the tubular connector. Other variants will be apparent to the skilled person.

Table 1 below contains exemplary dimensions for the implant.

**Table 1**

| Membrane thickness | Membrane Diameter | Bristle Diameter |
|---|---|---|
| 4 - 35 microns | 6.5mm | 0.381mm (0.015") |
| 4 - 35 microns | 9mm | 0.445mm (0.0175") |
| 4 - 35 microns | 16mm | 0.508mm (0.02") |

All of the above are fully within the scope of the present disclosure, and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## Claims

1. An embolisation device (100) for promoting clot formation in a bodily lumen (170), comprising a stem (110) and a plurality of flexible bristles (120) extending outwardly from the stem (110), the bristles (120) having a contracted delivery configuration and an expanded deployed configuration in which the bristles (120) extend at least radially outwardly from the stem (110) to anchor the device in the lumen (170), wherein the embolisation device (100) further comprises:
a flow restrictor configured to restrict flow through the bodily lumen (170), the flow restrictor comprising:
a membrane support (135) having a contracted delivery configuration and an expanded deployed configuration; and
an occluding mesh membrane (130) mounted on the membrane support (135), wherein the occlusion rate of the occluding mesh membrane (130) is greater than that of the membrane support (135);
wherein the flow restrictor is attached to the stem (110) at a first longitudinal end, the flow restrictor further comprising an open second longitudinal end, and
wherein the occluding mesh membrane (130) is configured to restrict flow through the lumen (170) when the device is deployed and the membrane support (135) is in the expanded deployed configuration;
**characterised in that**
the membrane support (135) comprises a self-expanding mesh extending at least radially outwardly from the stem (110).

2. An embolisation device (100) according to claim 1, wherein the occluding mesh membrane (130) is a metallic mesh membrane, preferably a Nitinol mesh membrane.

3. An embolisation device (100) according to claim 1 or 2, wherein the self-expanding mesh is attached to the stem (110) at a first longitudinal end, the self-expanding mesh further comprising a tubular section extending longitudinally along the stem (110), wherein the tubular section is configured to contact the bodily lumen (170) in the expanded deployed configuration.

4. An embolisation device (100) according to any preceding claim, wherein the occluding mesh membrane (130) is mounted radially outside of the self-expanding mesh.

5. An embolisation device (100) according to any preceding claim, wherein the occluding mesh membrane (130) comprises a first network of wires having a first average diameter, and the self-expanding mesh comprises a second network of wires having a second average diameter, wherein the second average diameter is greater than the first average diameter.

6. An embolisation device (100) according to any preceding claim, wherein the occluding mesh membrane (130) comprises a plurality of pores, and wherein the number of pores per unit area of the occluding mesh membrane (130) is greater than the number of pores per unit area of the self-expanding mesh.

7. An embolisation device (100) according to any preceding claim, wherein the occluding mesh membrane (130) comprises a plurality of pores having a lateral extent of less than 200µm, and preferably less than 100µm.

8. An embolisation device (100) according to any preceding claim, further comprising a tubular connector (300) receiving the stem (110) and the membrane support (135) to attach the membrane support (135) to the stem (110).

9. An embolisation device (100) according to claim 8, wherein the occluding mesh membrane (130) is attached to the stem (110) by the tubular connector (300).

10. An embolisation device (100) according to claims 8 or 9, the tubular connector (300) is a crimping connector.

11. An embolisation device (100) according to any preceding claim, wherein the occluding mesh membrane (130) is attached to the membrane support (135) by adhesive or welding.

12. An embolisation device (100) according to any preceding claim, wherein the flow restrictor is spaced apart from the plurality of bristles (120) such that in the expanded deployed configuration the flow restrictor is free from contact with any bristles (120), optionally wherein: the embolisation device (100) is configured to be delivered from a delivery catheter (160) to the bodily lumen (170) in a distal direction, and comprises a bristle segment (120b) positioned distally from the flow restrictor.

13. An embolisation device (100) according to any one of claims 1 to 11, wherein the embolisation device (100) comprises a bristle segment (120a) and the flow restrictor is located longitudinally within the bristle segment (120a) or directly adjacent the bristle segment (120a), optionally wherein:
the embolisation device (100) is configured to be delivered from a delivery catheter (160) to the bodily lumen (170) in a distal direction, the bristle segment (120a) is a proximal bristle segment and the embolisation device (100) further comprises a distal bristle segment (120b) spaced apart in the distal direction from the proximal bristle segment (120a).

14. A method of manufacturing an embolisation device (100) for promoting clot formation in a bodily lumen (170), comprising:
providing a stem (110);
providing a plurality of flexible bristles (120) extending outwardly from the stem (110), the bristles (120) having a contracted delivery configuration and an expanded deployed configuration in which the bristles (120) extend at least radially outwardly from the stem (110) to anchor the device in the lumen (170);
providing a membrane support (135) having a contracted delivery configuration and an expanded deployed configuration;
mounting an occluding mesh membrane (130) on the membrane support (135), the occluding mesh membrane (130) having a greater occlusion rate than that of the membrane support (135); and
attaching the membrane support (135) to the stem (110) such that it extends at least radially outwardly from the stem (110), the membrane support (135) having a contracted delivery configuration and an expanded deployed configuration in which the radial profile of the membrane support (135) is greater than in the contracted delivery configuration; wherein
the membrane support (135) is attached to the stem (110) at a first longitudinal end, the membrane support (135) further comprising an open second longitudinal end, and
the occluding mesh membrane (130) is configured to restrict flow through the lumen (170) when the device is deployed and the membrane support (135) is in the expanded deployed configuration;
**characterised in that** the membrane support (135) comprises a self-expanding mesh.

15. The method of claim 14, wherein the steps of providing the membrane support (135), mounting the occluding mesh membrane (130) on the membrane support (135) and attaching the membrane support (135) to the stem (110) comprise the steps of:
providing a self-expanding tubular mesh;
wrapping the occluding mesh membrane (130) around, and attaching it to, the tubular mesh;
providing a tubular connector (300) on the stem (110);
inserting an end of the tubular mesh into the tubular connector (300); and
crimping the connector (300),
optionally wherein:
the occluding mesh membrane (130) is wrapped around and attached to the tubular mesh before the tubular mesh is connected to the stem (110) by the crimping connector (300).

## Patentansprüche

1. Embolisationsvorrichtung (100) zur Förderung der Gerinnselbildung in einem Körperlumen (170), umfassend einen Stiel (110) und eine Vielzahl von flexiblen Borsten (120), die sich von dem Stiel (110) nach außen erstrecken, wobei die Borsten (120) eine kontrahierte Abgabekonfiguration und eine expandierte Einsatzkonfiguration aufweisen, in der sich die Borsten (120) zumindest radial nach außen von dem Stiel (110) erstrecken, um die Vorrichtung in dem Lumen (170) zu verankern, wobei die Embolisationsvorrichtung (100) weiter Folgendes umfasst:
einen Durchflussbegrenzer, der ausgebildet ist, um einen Durchfluss durch das Körperlumen (170) zu begrenzen, wobei der Durchflussbegrenzer Folgendes umfasst:
einen Membranträger (135), der eine kontrahierte Abgabekonfiguration und eine expandierte Einsatzkonfiguration aufweist;
und
eine okkludierende Netzmembran (130), die an dem Membranträger (135) montiert ist, wobei die Okklusionsrate der okkludierenden Netzmembran (130) größer ist als die des Membranträgers (135);
wobei der Durchflussbegrenzer an einem ersten Längsende an dem Stiel (110) befestigt ist, wobei der Durchflussbegrenzer weiter ein offenes zweites Längsende umfasst, und
wobei die okkludierende Netzmembran (130) ausgebildet ist, um einen Durchfluss durch das Lumen (170) zu begrenzen, wenn die Vorrichtung eingesetzt ist und der Membranträger (135) in der expandierten Einsatzkonfiguration ist;
**dadurch gekennzeichnet, dass**
der Membranträger (135) ein selbstexpandierendes Netz umfasst, das sich zumindest radial nach außen von dem Stiel (110) erstreckt.

2. Embolisationsvorrichtung (100) nach Anspruch 1, wobei die okkludierende Netzmembran (130) eine metallische Netzmembran, vorzugsweise eine Nitinol-Netzmembran, ist.

3. Embolisationsvorrichtung (100) nach Anspruch 1 oder 2, wobei das selbstexpandierende Netz an einem ersten Längsende an dem Stiel (110) befestigt ist, wobei das selbstexpandierende Netz weiter einen röhrenförmigen Abschnitt umfasst, der sich in Längsrichtung entlang des Stiels (110) erstreckt, wobei der röhrenförmige Abschnitt ausgebildet ist, um das Körperlumen (170) in der expandierten Einsatzkonfiguration zu kontaktieren.

4. Embolisationsvorrichtung (100) nach einem vorstehenden Anspruch, wobei die okkludierende Netzmembran (130) radial außerhalb des selbstexpandierenden Netzes montiert ist.

5. Embolisationsvorrichtung (100) nach einem vorstehenden Anspruch, wobei die okkludierende Netzmembran (130) ein erstes Netzwerk von Drähten, die einen ersten durchschnittlichen Durchmesser aufweisen, umfasst und das selbstexpandierende Netz ein zweites Netzwerk von Drähten, die einen zweiten durchschnittlichen Durchmesser aufweisen, umfasst, wobei der zweite durchschnittliche Durchmesser größer ist als der erste durchschnittliche Durchmesser.

6. Embolisationsvorrichtung (100) nach einem vorstehenden Anspruch, wobei die okkludierende Netzmembran (130) eine Vielzahl von Poren umfasst, und wobei die Anzahl der Poren pro Einheitsfläche der okkludierenden Netzmembran (130) größer ist als die Anzahl der Poren pro Einheitsfläche des selbstexpandierenden Netzes.

7. Embolisationsvorrichtung (100) nach einem vorstehenden Anspruch, wobei die okkludierende Netzmembran (130) eine Vielzahl von Poren, die eine seitliche Erstreckung von weniger als 200 µm, und bevorzugt weniger als 100 µm, aufweisen, umfasst.

8. Embolisationsvorrichtung (100) nach einem vorstehenden Anspruch, weiter umfassend einen röhrenförmigen Verbinder (300), der den Stiel (110) und den Membranträger (135) aufnimmt, um den Membranträger (135) an dem Stiel (110) zu befestigen.

9. Embolisationsvorrichtung (100) nach Anspruch 8, wobei die okkludierende Netzmembran (130) durch den röhrenförmigen Verbinder (300) an dem Stiel (110) befestigt ist.

10. Embolisationsvorrichtung (100) nach Anspruch 8 oder 9, wobei der röhrenförmige Verbinder (300) ein Quetschverbinder ist.

11. Embolisationsvorrichtung (100) nach einem vorstehenden Anspruch, wobei die okkludierende Netzmembran (130) durch Kleben oder Schweißen an dem Membranträger (135) befestigt ist.

12. Embolisationsvorrichtung (100) nach einem vorstehenden Anspruch, wobei der Durchflussbegrenzer von der Vielzahl von Borsten (120) beabstandet ist, so dass der Durchflussbegrenzer in der expandierten Einsatzkonfiguration keinen Kontakt mit jeglichen Borsten (120) hat, optional wobei: die Embolisationsvorrichtung (100) ausgebildet ist, um von einem Abgabekatheter (160) in das Körperlumen (170) in einer distalen Richtung abgegeben zu werden, und ein Borstensegment (120b) umfasst, das distal von dem Durchflussbegrenzer positioniert ist.

13. Embolisationsvorrichtung (100) nach einem der Ansprüche 1 bis 11, wobei die Embolisationsvorrichtung (100) ein Borstensegment (120a) umfasst und der Durchflussbegrenzer in Längsrichtung innerhalb des Borstensegments (120a) oder direkt benachbart zu dem Borstensegment (120a) angeordnet ist, optional wobei:
die Embolisationsvorrichtung (100) ausgebildet ist, um von einem Abgabekatheter (160) in das Körperlumen (170) in einer distalen Richtung abgegeben zu werden, das Borstensegment (120a) ein proximales Borstensegment ist und die Embolisationsvorrichtung (100) weiter ein distales Borstensegment (120b) umfasst, das in der distalen Richtung von dem proximalen Borstensegment (120a) beabstandet ist.

14. Verfahren zur Herstellung einer Embolisationsvorrichtung (100) zur Förderung der Gerinnselbildung in einem Körperlumen (170), umfassend:
Bereitstellen eines Stiels (110);
Bereitstellen einer Vielzahl von flexiblen Borsten (120), die sich von dem Stiel (110) nach außen erstrecken, wobei die Borsten (120) eine kontrahierte Abgabekonfiguration und eine expandierte Einsatzkonfiguration aufweisen, in der sich die Borsten (120) zumindest radial nach außen von dem Stiel (110) erstrecken, um die Vorrichtung in dem Lumen (170) zu verankern;
Bereitstellen eines Membranträgers (135), der eine kontrahierte Abgabekonfiguration und eine expandierte Einsatzkonfiguration aufweist;
Montieren einer okkludierenden Netzmembran (130) auf dem Membranträger (135), wobei die okkludierende Netzmembran (130) eine größere Okklusionsrate aufweist als die des Membranträgers (135); und
Befestigen des Membranträgers (135) an dem Stiel (110) derart, dass er sich zumindest radial nach außen von dem Stiel (110) erstreckt, wobei der Membranträger (135) eine kontrahierte Abgabekonfiguration und eine expandierte Einsatzkonfiguration aufweist, in der das radiale Profil des Membranträgers (135) größer ist als in der kontrahierten Abgabekonfiguration; wobei
der Membranträger (135) an einem ersten Längsende an dem Stiel (110) befestigt ist, wobei der Membranträger (135) weiter ein offenes zweites Längsende umfasst, und
die okkludierende Netzmembran (130) ausgebildet ist, um einen Durchfluss durch das Lumen (170) zu begrenzen, wenn die Vorrichtung eingesetzt ist und der Membranträger (135) in der expandierten Einsatzkonfiguration ist;
**dadurch gekennzeichnet, dass** der Membranträger (135) ein selbstexpandierendes Netz umfasst.

15. Verfahren nach Anspruch 14, wobei die Schritte des Bereitstellens des Membranträgers (135), des Montierens der okkludierenden Netzmembran (130) auf dem Membranträger (135) und des Befestigens des Membranträgers (135) an dem Stiel (110) die folgenden Schritte umfassen
Bereitstellen eines selbstexpandierenden röhrenförmigen Netzes;
Wickeln der okkludierenden Netzmembran (130) um das röhrenförmige Netz und befestigen derselben an diesem;
Bereitstellen eines röhrenförmigen Verbinders (300) auf dem Stiel (110);
Einführen eines Endes des röhrenförmigen Netzes in den röhrenförmigen Verbinder (300); und
Quetschen des Verbinders (300),
optional wobei:
die okkludierende Netzmembran (130) um das röhrenförmige Netz gewickelt und daran befestigt wird, bevor das röhrenförmige Netz durch den Quetschverbinder (300) mit dem Stiel (110) verbunden wird.

## Revendications

1. Dispositif d'embolisation (100) destiné à favoriser la formation de caillot dans une lumière corporelle (170), comprenant une tige (110) et une pluralité de poils flexibles (120) s'étendant vers l'extérieur à partir de la tige (110), les poils (120) ayant une configuration de distribution contractée et une configuration déployée d'extension dans laquelle les poils (120) s'étendent au moins radialement vers l'extérieur à partir de la tige (110) afin d'ancrer le dispositif dans la lumière (170), dans lequel le dispositif d'embolisation (100) comprend en outre :
un limiteur de débit configuré pour limiter le débit à travers la lumière corporelle (170), le limiteur de débit comprenant :
un support de membrane (135) ayant une configuration de distribution contractée et une configuration déployée d'extension ;
et
une membrane maillée occlusive (130) montée sur le support de membrane (135), dans lequel le débit d'occlusion de la membrane maillée occlusive (130) est supérieur à celui du support de membrane (135) ;
dans lequel le limiteur de débit est fixé à la tige (110) au niveau d'une première extrémité longitudinale, le limiteur de débit comprenant en outre une seconde extrémité longitudinale ouverte, et
dans lequel la membrane maillée occlusive (130) est configurée pour limiter le débit à travers la lumière (170) lorsque le dispositif est déployé et le support de membrane (135) est dans la configuration déployée d'extension ;
**caractérisé en ce que**
le support de membrane (135) comprend une maille auto-déployable s'étendant au moins radialement vers l'extérieur à partir de la tige (110).

2. Dispositif d'embolisation (100) selon la revendication 1, dans lequel la membrane maillée occlusive (130) est une membrane maillée métallique, de préférence une membrane maillée Nitinol.

3. Dispositif d'embolisation (100) selon la revendication 1 ou la revendication 2, dans lequel la maille auto-déployable est fixée à la tige (110) au niveau d'une première extrémité longitudinale, la maille auto-déployable comprenant en outre une section tubulaire s'étendant longitudinalement le long de la tige (110), dans lequel la section tubulaire est configurée pour venir au contact de la lumière corporelle (170) dans la configuration déployée d'extension.

4. Dispositif d'embolisation (100) selon une quelconque revendication précédente, dans lequel la membrane maillée occlusive (130) est montée radialement à l'extérieur de la maille auto-déployable.

5. Dispositif d'embolisation (100) selon une quelconque revendication précédente, dans lequel la membrane maillée occlusive (130) comprend un premier réseau de fils ayant un premier diamètre moyen, et la maille auto-déployable comprend un second réseau de fils ayant un second diamètre moyen, dans lequel le second diamètre moyen est supérieur au premier diamètre moyen.

6. Dispositif d'embolisation (100) selon une quelconque revendication précédente, dans lequel la membrane maillée occlusive (130) comprend une pluralité de pores, et dans lequel le nombre de pores par surface unitaire de la membrane maillée occlusive (130) est supérieur au nombre de pores par surface unitaire de la maille auto-déployable.

7. Dispositif d'embolisation (100) selon une quelconque revendication précédente, dans lequel la membrane maillée occlusive (130) comprend une pluralité de pores ayant une étendue latérale inférieure à 200 µm, et de préférence inférieure à 100 µm.

8. Dispositif d'embolisation (100) selon une quelconque revendication précédente, comprenant en outre un connecteur tubulaire (300) recevant la tige (110) et le support de membrane (135) afin de fixer le support de membrane (135) à la tige (110).

9. Dispositif d'embolisation (100) selon la revendication 8, dans lequel la membrane maillée occlusive (130) est fixée à la tige (110) par le connecteur tubulaire (300).

10. Dispositif d'embolisation (100) selon les revendications 8 ou 9, le connecteur tubulaire (300) est un connecteur à sertir.

11. Dispositif d'embolisation (100) selon une quelconque revendication précédente, dans lequel la membrane maillée occlusive (130) est fixée au support de membrane (135) par adhésif ou soudage.

12. Dispositif d'embolisation (100) selon une quelconque revendication précédente, dans lequel le limiteur de débit est espacé de la pluralité de poils (120) de telle sorte que dans la configuration déployée d'extension le limiteur de débit soit exempt de venir au contact des poils (120), facultativement dans lequel : le dispositif d'embolisation (100) est configuré pour être distribué depuis un cathéter de distribution (160) jusqu'à la lumière corporelle (170) dans une direction distale et comprend un segment de poil (120b) positionné distalement par rapport au limiteur de débit.

13. Dispositif d'embolisation (100) selon l'une quelconque des revendications 1 à 11, dans lequel le dispositif d'embolisation (100) comprend un segment de poil (120a) et le limiteur de débit est situé longitudinalement à l'intérieur du segment de poil (120a) ou directement adjacent au segment de poil (120a), facultativement dans lequel :
le dispositif d'embolisation (100) est configuré pour être distribué depuis un cathéter de distribution (160) jusqu'à la lumière corporelle (170) dans une direction distale, le segment de poil (120a) est un segment de poil proximal et le dispositif d'embolisation (100) comprend en outre un segment de poil distal (120b) espacé dans la direction distale par rapport au segment de poil proximal (120a).

14. Procédé destiné à fabriquer un dispositif d'embolisation (100) pour favoriser la formation de caillot dans une lumière corporelle (170), comprenant :
fournir une tige (110) ;
fournir une pluralité de poils flexibles (120) s'étendant vers l'extérieur à partir de la tige (110), les poils (120) ayant une configuration de distribution contractée et une configuration déployée d'extension dans laquelle les poils (120) s'étendent au moins radialement vers l'extérieur à partir de la tige (110) afin d'ancrer le dispositif dans la lumière (170) ;
fournir un support de membrane (135) ayant une configuration de distribution contractée et une configuration déployée d'extension ;
monter une membrane maillée occlusive (130) sur le support de membrane (135), la membrane maillée occlusive (130) ayant un débit d'occlusion supérieur à celui du support de membrane (135) ; et
fixer le support de membrane (135) à la tige (110) de telle sorte qu'il s'étende au moins radialement vers l'extérieur à partir de la tige (110), le support de membrane (135) ayant une configuration de distribution contractée et une configuration déployée d'extension dans laquelle le profil radial du support de membrane (135) est supérieur à celui dans la configuration de distribution contractée ; dans lequel
le support de membrane (135) est fixé à la tige (110) au niveau d'une première extrémité longitudinale, le support de membrane (135) comprenant en outre une seconde extrémité longitudinale ouverte, et
la membrane maillée occlusive (130) est configurée pour limiter le débit à travers la lumière (170) lorsque le dispositif est déployé et que le support de membrane (135) est dans la configuration déployée d'extension ;
**caractérisé en ce que** le support de membrane (135) comprend une maille auto-déployable.

15. Procédé selon la revendication 14, dans lequel les étapes destinées à fournir le support de membrane (135), monter la membrane maillée occlusive (130) sur le support de membrane (135) et fixer le support de membrane (135) à la tige (110) comprennent les étapes destinées à
fournir une maille tubulaire auto-déployable ;
enrouler la membrane maillée occlusive (130) autour de la maille tubulaire et la fixer à celle-ci ;
fournir un connecteur tubulaire (300) sur la tige (110) ;
insérer une extrémité de la maille tubulaire dans le connecteur tubulaire (300) ; et
sertir le connecteur (300),
facultativement dans lequel :
la membrane maillée occlusive (130) est enroulée autour de la maille tubulaire et fixée à celle-ci avant que la maille tubulaire soit reliée à la tige (110) par le connecteur à sertir (300).
